(19) Europäisches Patentamt
European Patent Office
Office européen des brevets

(11) **EP 1 928 407 B1**

(12) **EUROPEAN PATENT SPECIFICATION**

(45) Date of publication and mention of the grant of the patent:
**18.03.2009 Bulletin 2009/12**

(51) Int Cl.:
*A61K 9/00* (2006.01)   *A61K 31/5575* (2006.01)
*A61K 31/535* (2006.01)

(21) Application number: **06779382.8**

(22) Date of filing: **11.09.2006**

(86) International application number:
**PCT/GB2006/003366**

(87) International publication number:
**WO 2007/034140 (29.03.2007 Gazette 2007/13)**

(54) **CHRONOTHERAPEUTIC OCULAR DELIVERY SYSTEM COMPRISING A COMBINATION OF PROSTAGLANDIN AND A BETA-BLOCKER FOR TREATING PRIMARY GLAUCOMA**

CHRONOTHERAPEUTISCHES OKULARES ABGABESYSTEM AUS EINER KOMBINATION VON PROSTAGLANDINEN UND EINEM BETABLOCKER ZUR BEHANDLUNG VON PRIMÄREM GLAUKOM

SYSTEME D'ADMINISTRATION OCULAIRE CHRONOTHERAPEUTIQUE COMPRENANT UNE COMBINAISON DE PROSTAGLANDINE ET D'UN BETABLOQUANT POUR LE TRAITEMENT D'UN GLAUCOME PRIMAIRE

(84) Designated Contracting States:
**AT BE BG CH CY CZ DE DK EE ES FI FR GB GR HU IE IS IT LI LT LU LV MC NL PL PT RO SE SI SK TR**

(30) Priority: **21.09.2005 GB 0519270**
**25.04.2006 GB 0608181**

(43) Date of publication of application:
**11.06.2008 Bulletin 2008/24**

(73) Proprietor: **Aston University**
**Birmingham B4 7ET (GB)**

(72) Inventors:
• **CONWAY, Barbara, R.**
**Birmingham**
**West Midlands B29 4AY (GB)**

• **GHERGHEL, Doina**
**West Midlands B73 5BS (GB)**

(74) Representative: **Bannerman, David Gardner et al**
**Withers & Rogers LLP**
**Goldings House**
**2 Hays Lane**
**London**
**SE1 2HW (GB)**

(56) References cited:
**WO-A-20/05110424**     **US-A- 6 051 576**
**US-A1- 2004 013 704**

**Description**

The present invention relates to a chronotherapeutic delivery system for the treatment of primary open-angle glaucoma which enables pharmaceutical products to be delivered to the eye in a controlled manner when required during the hours of sleep.

***Background of the invention,***

*Circadian rhythm of IOP*

Primary open-angle glaucoma (POAG) represents a chronic, slowly progressive optic neuropathy, characterized by progressive excavation of the optic nerve head (ONH) and a distinctive pattern of visual field (VF) defects. The disease is multifactorial in origin and is associated more closely with elevated intraocular pressure (IOP) resulting in the main from reduced drainage of aqueous humor. Glaucoma is generally managed by reducing a high IOP to a so called "target pressure"; however, this pressure is difficult to identify because IOP fluctuates throughout the day and night according to its circadian rhythm. Moreover, the finding that about one third of patients develop glaucoma while exhibiting apparently normal IOP during their daytime clinical appointments, or the fact that a substantial number of cases with POAG continue to progress despite therapeutically lowered IOP, has triggered more extensive investigations and development of new therapeutic strategies to control the progression of this disease.

A large variety of physiological functions have a circadian rhythm dependent on the autonomic nervous system (ANS). Among other functions, ANS also affects the aqueous humor (AH) dynamics and IOP. AH has a circadian rhythm with a higher rate of secretion during the day and a lower rate at night. Sleep deprivation at night is associated with an increase in AH flow compared to that during normal sleep; however, the aqueous flow does not rise to daytime levels showing that there is a true circadian rhythm that is independent of nocturnal waking. Circadian variations in AH secretion should result in similar changes in the level of IOP. Therefore, clinicians initially believed that the level of IOP was highest in the morning, lower later in the afternoon and lowest at night. This belief has had clinical consequences: IOP has been measured only during the day, especially in the morning while the patient attended the clinical examination and treatment has therefore been developed to address this particular IOP profile, using drugs that inhibit the AH production, such as beta-blockers. Later, however, it has been demonstrated that although in a significant number of glaucoma patients the 24-hour IOP values decrease during the night, in other normal subjects and patients with glaucoma the IOP curve had a different shape. IOP has been found to be higher during sleep by a number of studies performed 10-20 years ago. The increase was sharp at the onset of sleep in young subjects and gradual, throughout the night in elderly. Similar recent results confirm that night time IOP was higher than daytime IOP in both young and elderly subjects. It has also been reported that glaucoma patients demonstrated a further increase in IOP between 5:30 and 7:30 in the morning, while normal subjects experienced a decrease in IOP; the authors concluded that regulation of IOP in glaucoma patients was different from that seen in healthy subjects.

This finding could suggest that IOP has a true circadian rhythm and any disturbances of this endogenous circadian rhythm could play an important role in the pathogenesis of glaucoma. However, a more important conclusion could be that treatments addressed to decreasing AH production could not be beneficial for some patients with high nocturnal IOP levels. Indeed, it has been demonstrated that timolol had a reduced capacity in decreasing IOP at night. Agents that increase uveo-scleral outflow (more potent at night) of the AH, such as the prostaglandin analogues (e.g. latanoprost, bimatoprost and travoprost) are, in this respect, more efficient in decreasing the 24-h IOP. However, even these pharmaceutical products have shown decreased efficiency towards lowering the IOP during early hours of the morning. Moreover, dangerous IOP peaks and IOP fluctuations occur outside of normal office hours; these findings are more significant in patients with disease progression despite proper use of their medication.

*Circadian rhythm of systemic and ocular circulation*

Other risk factors have also been associated with the occurrence and progression of POAG. One of the most studied routes is the investigation of the role of various systemic and ocular circulatory deficiencies in the etiology of glaucoma. Among factors that may influence the blood flow physiology, variables such as BP and heart rate (HR) also have a circadian rhythm dependent on the ANS. Systemic BP for example, has a circadian rhythm characterized by a physiologic nocturnal dip in BP (representing the fall in blood pressure during night time expressed as a percentage of the average daytime reading level) of around 10% to 20%, which is present in approximately two thirds of the healthy population (known as dippers). Non-dippers have a nocturnal BP fall of less than 10%, and are characterized by increased frequency of myocardial ischemia, cerebrovascular damage including stroke, haemorrhages, thrombosis and vascular dementia, possibly because these patients suffer a longer duration of exposure to high BP levels over 24 hours. The so-called extreme dippers have a nocturnal fall in BP of more than 20%, which may occur naturally or due to the use of antihypertensive medications. These patients could also exhibit ischemic phenomena, including cardiac ischemia, silent cerebrovascular damage, and anterior ischemic optic neuropathy (AION). It has been shown that the frequency of large blood pressure dips in either progressive open-angle glaucoma or NTG was higher than in POAG patients with stable visual field defects or normal controls. The importance of low nocturnal BP values in patients with both NTG and progressive POAG has also been demonstrated.

There is little doubt that low blood pressure and especially a nocturnal over-dip is an essential risk factor for POAG of similar importance to the risk from increased IOP. In this regard, the investigation of IOP peaks, as has been long-standing practice, should be matched by the search for nocturnal BP dips, as either or both of these may result in altered ocular hemodynamics, with resultant damage in susceptible patients.

The non-dipping phenomenon has been closely related to a profound autonomic dysfunction and to a blunted endothelium-dependent vasodilation through a decreased nitric oxide (NO) release. NO was found to be the major determinant of cerebral blood flow differences that exist between sleep-wake states and to contribute to the basal retinal vascular tone. Moreover, NO also modulates IOP and any disturbance in the NO balance acts both locally, at the ocular level, and systemically. Therefore, a low NO production has important consequences on the equilibrium between the endothelial vasoconstrictory and vasodilatory factors; this can result in a decreased ocular blood flow (OBF) in susceptible patients. In association with a high IOP during early hours of the morning, this effect has dramatic consequences on the progression of POAG.

In susceptible patients, a high nocturnal IOP could occur in association with either an exaggerated dip in systemic BP and/or insufficient OBF; this combination could result in poor disease control in some glaucoma patients. As previously stated, the current available antiglaucomatous drops have a good 24-hour IOP control but unwanted IOP spikes still occur, especially during early hours of the morning. During this time, the patient is also susceptible to systemic and ocular circulatory events that could affect even further the progression of the disease. A drug/delivery system with double action (a uniform 24-h IOP control, together with an OBF improvement capacity, especially during early hours of the morning) will have better benefits that the currently available antiglaucomatous medication in controlling glaucoma in those cases, in which the occurrence of the disease and/or disease progression is due to multiple risk factors. This represents a first chronotherapeutic step in glaucoma management.

### Chronotherapy

In medicine, chronotherapy is used in a number of diseases such as systemic hypertension, cardiac ischaemic diseases and asthma. A chronotherapeutic agent represents a pharmaceutical product that contains a dynamic element such as a delivery system. Therefore, the drug is delivered at the time when it is needed.

### Pulsatile delivery systems

For subcutaneous implants, delivery of the pharmaceutical product can be regulated in bursts separated by dormant intervals with little or no delivery. Pulsatile delivery can be achieved using a stimuli-responsive system whereby a change in the local environment triggers the delivery of the pharmaceutical product. Triggers include temperature used to sustain delivery of anti-glaucoma agents from polymeric eye drops and iontophoresis has been used to trigger delivery of gentamicin to the eye. However, for the novel approach to glaucoma therapy according to the invention, the peak in delivery is effected while the patient is asleep. Such known systems are not suitable for this application. Another known method of achieving a pulsatile delivery is to use a pre-programmed delivery system where delivery of the pharmaceutical product is controlled by the design of the system itself. This has been achieved using a cylindrical laminate formulation, with layers of pharmaceutical product-containing polyphosphazene polymers and pharmaceutical product-free polyanhydride spacers and a biodegradable hydrophobic coating. Using alternating core layers, the lag time and duration of delivery are specified for subcutaneous implantation.

### Ocular delivery systems

In ophthalmic therapy, a number of solid polymeric inserts and discs have been developed as ocular delivery systems. They are better tolerated as to drainage and tear flow compared with other ophthalmic formulations and produce reliable delivery in the conjunctival cul-de-sac. They are also believed to reduce systemic side-effects and require less frequent administration. Known controlled delivery systems designed to provide a continuous delivery include ocular inserts, minitablets, disposable lenses and ocular films.

Previously, it has been believed that a slow, zero order delivery rate is the ideal for anti-glaucoma deliveries from inserts such as the delivery provided by Ocusert® or Ocufit®. Ocusert® Pilo (Alza Corporation) consists of a delivery reservoir, pilocarpine HCl in an alginate gel, enclosed by two delivery-controlling membranes made of ethylene-vinyl acetate copolymer and enclosed by a white retaining ring impregnated with titanium oxide, allowing positioning of the system in the eye. Lacrisert® (a hydroxypropyl cellulose ophthalmic insert) is a sterile, translucent, rod-shaped, water-soluble, ophthalmic insert made of hydroxypropyl cellulose, for administration into the inferior cul-de-sac of the eye. A fluorescent marker has been delivered from a compressed formulation containing Carbopol (a known polyacrylic acid mucoadhesive polymer). Delivery was extended for up to eight hours using a highly compressed minitablet with slow hydration rate. Fluorescein has also been successfully incorporated into polyacrylic acid-cysteine inserts which have shown a sustained delivery in humans beyond the eight hours estimated in vitro and were well tolerated. Diclofenac sodium, an anti-inflammatory drug, was incorporated into the inserts and showed prolonged release in vitro.

US2004013704 discloses an intraocular implant comprising a lipophilic compound and a combination of ophthalmic drugs, such as combinations of a beta-blocker (betaxolol and timolol) and prostaglandin (latanoprost, 15-keto latanoprost, fluprostenol isopropyl ester). In US6051576 a sustained release, and substantially inactive codrug is described, which comprises at least two drugs ionically or covalently linked to one another, which itself is fixed to an implantable device. As a specific example prostaglandin F2 alpha was given, which is covalently linked to form a codrug with timolol.

## Brief Description of the Drawings

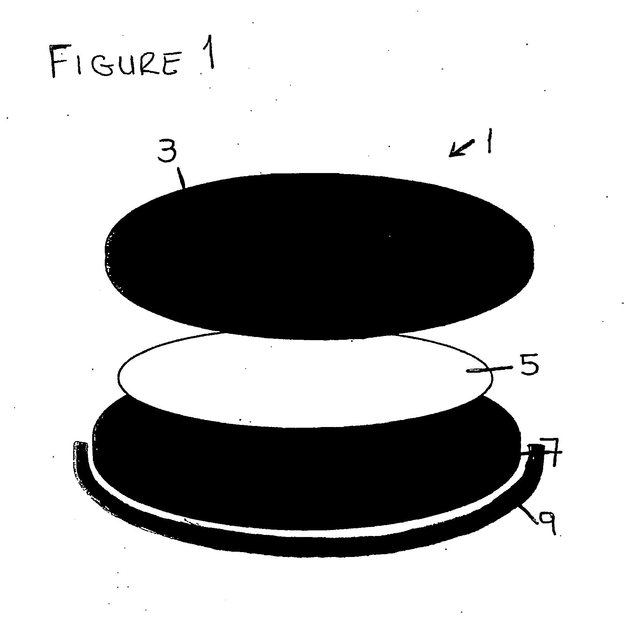

Figure 1.     This shows a design for chronotherapeutic ocular insert including a biodegradable polymer layer.

Figure 2.     This shows a percentage release profile of latanoprost and betaxolol using a delivery system according to the invention.

Figure 3.     This shows a percentage release profile of latanoprost and timolol using a delivery system according to the invention.

Figure 4.     This shows a percentage release profile of travoprost from an ocular insert.

Figure 5.     This shows a percentage release profile of travoprost and timolol using a delivery system according to the invention.

*Design of a chronotherapeutic ocular insert*

Although there are many systems, both marketed and in development, for zero order release, methods for achieving pulsatile delivery of pharmaceutical products in ocular formulations have not been developed until now. There has therefore been a need for an ocular delivery system designed to release its load at a predetermined time. The present invention makes it possible to maintain a constant delivery of a prostaglandin analogue from an erodible material with delivery of a beta-blocker once the material has eroded sufficiently to permit water uptake.

Therefore, in accordance with the present invention, there is provided a delivery system (1) incorporating pharmaceutical products for delivery to an eye in the treatment of primary open-angle glaucoma (POAG), comprising:

(a) a biocompatible erodible material (3) incorporating a therapeutically-effective amount of a prostaglandin analogue; and

(b) a reservoir (7) containing a therapeutically-effective amount of a beta-blocker,
whereby, when the delivery system is placed in the eye, the prostaglandin analogue is delivered gradually as the erodible material is eroded, and the beta-blocker is delivered rapidly when at least a predetermined portion, and preferably substantially all, of the erodible material has been eroded.

Use of an ocular insert reduces the risk of loss of drug from the eye so that a lower dose of beta-blocker will be effective, thus reducing the likelihood of unwanted systemic effects of the beta-blocker, such as lowering of blood pressure.

Erodible inserts composed of polyethylene oxide have previously been tested for delivery of antibiotics where delivery was controlled by polymer swelling or erosion or delivery diffusion through the hydrated gel. With a soluble insert such as a polyvinyl alcohol film (NODS), increased bioavailability of pilocarpine has been shown but the degree of control over the delivery process is not adequate for a delayed pulsatile release on its own. PVA films have a half-life of approximately eight minutes so once this level is reached, delivery is rapid. This type of soluble insert may be used to form the reservoir for the beta-blocker and to provide a rapid delivery of the beta-blocker such as betaxolol at the programmed time while a layer of an erodible material provides delivery of the prostaglandin analogue, examples of which are latanoprost which is a lipophilic prostaglandin analogue prodrug with enhanced corneal penetration, bimatoprost, which is a synthetic fatty acid amide analogue, travoprost, and unoprostone isopropyl. The half-life of the active ingredient of latanoprost, latanoprost acid, is 2.8 hours in aqueous humor and both latanoprost and bimatoprost are designed for once daily application. Preferred beta-blockers are betaxolol and timolol maleate. Betaxolol is formulated as the hydrochloride salt and onset of action can generally be noted within about 30 minutes and the maximum effect can usually be detected about 2 hours after topical administration.

Preferably, the delivery system is an insert which can be placed in the eye at night, with the erodible material slowly eroding, causing a controlled delivery of the prostaglandin analogue followed by a rapid (or pulsatile) delivery of the beta-blocker after a delayed phase to coincide with periods of high IOP and low OBF in a patient. Preferably, the delivery of the beta-blocker occurs when substantially all of the erodible material has been eroded. "Pulsatile" means rapid delivery after a period of essentially no delivery. As this insert is intended to be placed in the eye at night, a time-controlled component is the preferred mode of delivery.

The insert preferably consists of an erodible material and a mucoadhesive component that will lead to formation of a superficial gel. Inadvertent loss of the delivery system from the eye during sleep may be minimised by incorporation of the mucoadhesive component. A preferred mucoadhesive component is a polyacrylic acid polymer, such as Carbopol®, which is incorporated in an amount of up to about 15% by weight of the erodible material, preferably from 5-12% by weight, more preferably about 10% by weight. The erosion kinetics determine the timing of the pulsatile delivery and will be understood by one skilled in the technical field. The erodible material preferably comprises a polymeric material. A number of polymers can be used for controlled delivery of the prostaglandin analogue including polyethylene oxide (PEO), co-polymers of methyl vinyl ether and maleic anhydride (PVM/MA), poly(alkyl cyanoacrylates), polylactic and glycolic acid copolymers. The erosion of polyethylene oxide gel-forming inserts can be optionally modified by the inclusion of a methacrylic acid polymer such as Eudragit®, and the delivery can be controlled. However, it has been observed that the use of Carbopol® as the mucoadhesive component also has the added advantage in that it causes a greater percentage release of the prostaglandin from the erodible material without the need for any other control additives to be incorporated in the delivery system.

Naturally-occurring or synthetic materials which are biologically compatible with body fluids and eye tissues and substantially insoluble in body fluids with which the material will come into contact, and which are therefore suitable to be used for the reservoir to contain the beta-blocker, include, but are not limited to, glass, metal, ceramics, polyvinyl alcohol (PVA), cross-linked polyvinyl alcohol, polyvinyl acetate, polyvinylbutyrate, cross-linked polyvinyl butyrate, ethylene ethylacrylate copolymer, polyethyl hexylacrylate, polyvinyl chloride, polyvinyl acetals, plasticised ethylene vinylacetate copolymer, ethylene vinylchloride copolymer, polyvinyl esters, polyvinylformal, polyamides, polymethylmethacrylate, polybutylmethacrylate, plasticised polyvinyl chloride, plasticised nylon, plasticised soft nylon, plasticised polyethylene terephthalate, natural rubber, polyisoprene, polyisobutylene, polybutadiene, polyethylene, polytetrafluoroethylene, polyvinylidene chloride, polyacrylonitrile, cross-linked polyvinylpyrrolidone, polytrifluorochloroethylene, chlorinated polyethylene, poly(1,4'-isopropylidene diphenylene carbonate), vinylidene chloride, acrylonitrile copolymer, vinyl chloride-diethyl fumerale copolymer, butadiene/styrene copolymers, silicone rubbers, especially the medical grade polydimethylsiloxanes, ethylene-propylene rubber, silicone-carbonate copolymers, vinylidene chloride-vinyl chloride copolymer, vinyl chloride-acrylonitrile copolymer and vinylidene chloride-acrylonitride copolymer.

The reservoir may be formed from a non-biodegradable polymer. Such non-biodegradable polymers are well-known and may include, for example, polymethylmethacrylate, a silicone elastomer, or silicone rubber. Other suitable non-erodible, biocompatible polymers which may be used in fabricating the reservoir may include polyolefins such as polypropylene and polyethylene, homopolymers, and copolymers of vinyl acetate such as ethylene vinyl acetate copolymer, polyvinylchlorides, homopolymers and copolymers of acrylates such as polyethylmethacrylate, polyurethanes, polyvinylpyrrolidone, 2-pyrrolidone, polyacrylonitrile butadiene, polycarbonates, polyamides, fluoropolymers such as polytetrafluoroethylene and polyvinyl fluoride, polystyrenes, homopolymers and copolymers of styrene acrylonitrile, cellulose acetate, homopolymers and copolymers of acrylonitrile butadiene styrene, polymethylpentene, polysulfones, polyesters, polyimides, natural rubber, polyisobutylene and polymethylstyrene.

As a further alternative, the reservoir may be formed from a synthetic biodegradable polymer that can contain microparticles of the substance to be delivered. Thus, in this embodiment, as the polymer is eroded, the beta-blocker is delivered into the eye. Delivery time is a function of the polymer and the formulation. By way of example, some suitable biodegradable polymers include polyesters of molecular weight from about 4,000 to about 100,000, homopolymers and copolymers of (poly)lactic acid and (poly)glycolic acid, such as polylactic glycolic acid copolymer (PLGA), polycaprolactone, homopolymers and copolymers of polyanhydrides such as terephthalic acid anhydride, bis(p-anhydride) and poly(p-carboxyphenoxy) alkyl, homopolymers and copolymers of dicarboxylic acids such as sebacic, adipic, oxalic, phthalic and maleic acid, polymeric fatty acid dimer compounds such as polydodecanedioic acid polyorthoesters, poly (alkyl-2-cyanoacrylate) such as poly(hexyl-2-cyanoacrylate), collagen (gelatin), polyacetals, divinyloxyalkylenes, polydihydropyrans, polyphosphazenes, homopolymers and copolymers of amino acids such as copolymers of leucine and methyl glutamate, polydioxinones, polyalkylcyano acetates, polysaccharides and their derivatives such as dextran and cyclodextran, cellulose and hydroxymethyl cellulose.

Preferably, the reservoir comprises PVA.

The delivery system may be in the form of a laminate, in which case the two pharmaceutical products are located in separate layers. These two layers can be co-compressed to form a bi-layer.

The delivery system of the invention also preferably comprises a further layer of a biodegradable polymer 5 which is situated between the layer of erodible material and the reservoir. An example of such a biodegradable polymer which may be used in accordance with the invention is a polylactic glycolic acid copolymer (PLGA). In delivery systems including such a layer of a biodegradable polymer, at least a predetermined portion of the biodegradable polymer is biodegraded prior to the delivery of the beta-blocker.

According to a further embodiment of the invention, a membrane, such as a semi-permeable membrane, may be included to delay the delivery of the beta-blocker as an alternative to the biodegradable polymer. Again, the membrane can be co-compressed with the layers, or the membrane can be adhered to either layer. A preferred semi-permeable membrane is one comprising ethylcellulose.

The molecular weight of the polymers used as the erodible material and the biodegradable polymer is of importance with regard to the delivery profile of the prostaglandin and beta-blocker. For example, if the speed of delivery of the prostaglandin is measured using polyethylene oxides with molecular weights of about 100,000 and 400,000, little variation is observed. However, if the same measurement is carried .out using polyethylene oxides with molecular weights of about 400,000 and 900,000, the delivery of the prostaglandin is significantly slower with the polyethylene oxide having a molecular weight of about 900,000. With molecular weights greater than about 900,000, polymer swelling is too excessive to be of use as in ocular inserts. Therefore, if a slower delivery profile is required for a patient, then this can also be achieved by simple modification of the molecular weight of the polymer used as the erodible material. However, in the present invention, it is preferred that the molecular weight of the polymer used as the erodible material is between about 50,000 and about 600,000, more preferably between about 100,000 and about 500,000, and is most preferably about 400,000.

Similar results are observed when comparing different molecular weights of the biodegradable polymer; a molecular weight of less than 5,000 results in a more rapid delivery of the beta-blocker compared with molecular weights of about 5,000 to about 15,000, which in turn allows for a more rapid delivery than molecular weights of about 50,000 to about 75,000. Therefore, again, if a greater delay in the delivery of the beta-blocker is required for a patient, then this can also be achieved by simple modification of the molecular weight of the polymer used as the biodegradable polymer. However, in the present invention, it is preferred that the molecular weight of the polymer used as the biodegradable polymer is up to about 100,000, preferably up to about 75,000, more preferably up to about 50,000, still more preferably up to about 15,000, and most preferably up to about 5,000.

It has been observed that while a delivery system comprising a reservoir containing a beta-blocker as one of the layers is effective in delivering the necessary drugs to an eye, the delay in the release of the beta-blocker can be prolonged further to the optimum point in time. This can be achieved by, for example, supporting the sides of the reservoir with a non-permeable material to prevent premature leakage of the beta-blocker. A suitable material for this is polypropylene, which has previously been employed in ocular inserts.

The release profile of the delivery system according to the invention is further optimised by the incorporation of a barrier layer 9 of a biodegradable polymer or a non-biodegradable polymer located on the surface of the reservoir containing the beta-blocker opposite to the surface contacting the erodible material incorporating the prostaglandin analogue or the biodegradable polymer.

The prostaglandin analogue is preferably selected from latanoprost, bimatoprost, travoprost and unoprostone isopropyl.

The beta-blocker contained in the reservoir is preferably betaxolol or a salt thereof, or alternatively carteolol hydrochloride, levobunolol hydrochloride, metipranolol, or timolol maleate.

To delay the delivery, the beta-blocker may be encapsulated in a micro-particulate formulation.

In a most preferred embodiment, the delivery system of the invention comprises a layer of an erodible material containing a therapeutically-effective amount of a prostaglandin analogue, a layer of a biodegradable polymer, a reservoir layer containing a therapeutically-effective amount of a beta-blocker, wherein the sides of the reservoir layer are supported to inhibit leakage of the beta-blocker, and a further barrier layer located on the other side of the reservoir layer.

The prostaglandin analogue is most preferably latanoprost, while the beta-blocker is most preferably betaxolol. The erodible material is most preferably PEO, the biodegradable polymer is most preferably PLGA, the reservoir most preferably comprises PVA, and polypropylene is most preferably used to replace the sides of the reservoir layer. As the further barrier, any barrier may be used which effectively inhibits the loss of the beta-blocker from the delivery system. However, most preferred barriers are a further layer of PLGA or polypropylene. The most preferred mucoadhesive polymer is a polyacrylic polymer, such as Carbopol.

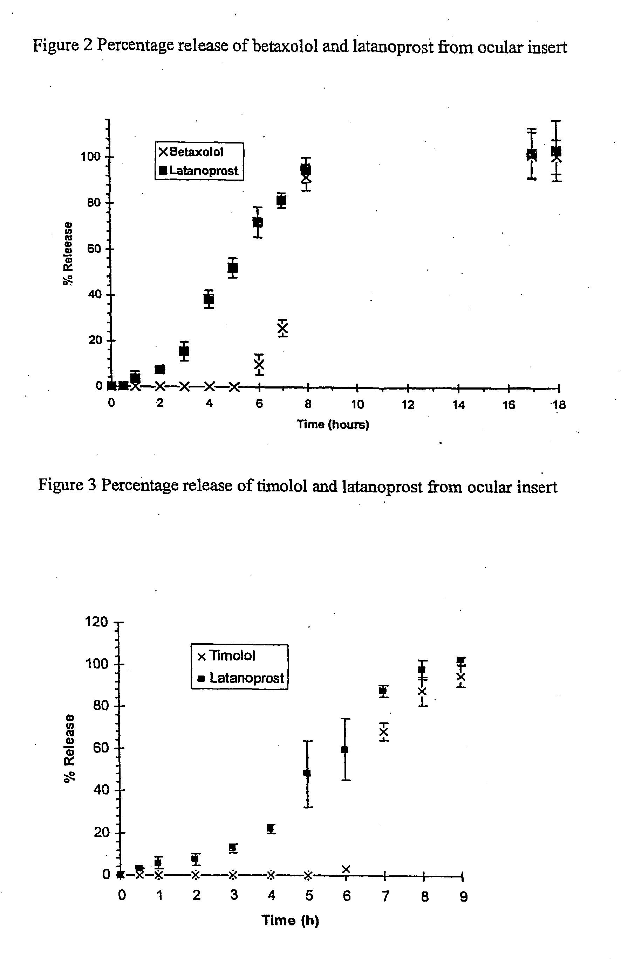

The use of such a structure optimises the time delay for the release of the beta-blocker and also enables the release to be accelerated such that near 100% delivery of the beta-blocker is achieved within about 2 to 3 hours of first release (see Figures 2 and 3).

Also envisaged within the present invention is a method of treating or preventing primary open-angle glaucoma in a mammal, comprising administering to the mammal a delivery system incorporating a prostaglandin analogue and a beta-blocker according to the invention, as well as the use of such a delivery system in such treatment.

According to a further embodiment of the invention, there is provided a process for the preparation of a delivery system incorporating a prostaglandin analogue and a beta-blocker according to the invention, comprising the steps of:

(a) dissolving a formulation containing the prostaglandin analogue in a solvent;

(b) adding this solution to the erodible material;

(c) removing the solvent;

(d) compressing the resultant powder into discs;

(e) dissolving a formulation containing the beta-blocker in a solvent;

(f) adding this solution to material comprising the reservoir;

(g) removing the solvent;

(h) compressing the resultant powder into discs;

(i) combining discs from steps (d) and (h).

The solvent may be any biologically-acceptable solvent which is capable of dissolving the formulation containing the prostaglandin analogue and beta-blocker.

Where a layer of a biodegradable polymer is to be included between the erodible material and the reservoir, the above method also includes the steps of grinding the biodegradable polymer into a fine powder, compressing the resultant powder into discs, and combining the resultant discs with those from from steps (d) and (h) above.

Non-limiting examples of the invention will now be described.

The inserts may also contain one or more of the following: surfactants, adjuvants including additional medicaments, buffers, antioxidants, tonicity adjusters, preservatives, thickeners or viscosity modifiers, and the like. Additives in the formulations may desirably include sodium chloride, EDTA (disodium edetate), and/or BAK (benzalkonium chloride), sorbic acid, methyl paraben, propyl paraben, chlorhexidine, and sodium perborate.

### Erodible Component

The first polymers assessed for the erodible component were polyethylene oxide of different molecular weights (supplied by Sigma). These are compressed into discs of approximately 300 $\mu$m thickness and the delivery to be sustained over 8 hours. These figures are comparable to BODI (5.0 x 2.0 mm; 20.5 mg weight) and SODI (9 x 4.5 mm x 0.35 mm 15-16 mg weight). Ocufit uses a rod shape which is said to be less easily lost from the eye-shape of Lacrisert (Merck).

### HPLC

One example of HPLC assays for both potential pharmaceutical products: latanoprost and betaxolol-HCl have been developed.

Purity was assigned as 100% as the same batch of pharmaceutical products which was used for the sample, and standard preparations throughout. Acetonitrile was supplied from Fisher (Loughborough, UK). Double-distilled water was produced in-house using a Fison's FiStreem still. All materials were of analytical, pharmaceutical or HPLC grade as appropriate.

### Equipment for quantification of latanoprost

The HPLC system comprised a Hewlet Packard Aligent 1100 system with G1312A Binary Pump, G1313A ALS Auto-injector, G1316A COLCOM column section and a G1314A VWD variable wavelength detector. The HPLC was controlled by Chemstation software which ran on Windows 2000. Standards 10 to 0.1mg/ml were diluted from 1500mg/ml stock solution (described below) in lachrymal fluid as required, and each run was 15 minutes. Chromatographic conditions are detailed in Table 1.

Table 1. Chromatographic conditions for detection of latanoprost

| Injection volume | 25 µl |
|---|---|
| Mobile phase | 60:40 acetonitrile: 0.1 % acetic acid in water |
| Flow rate | 1 ml/min |
| Wavelength | 210 nm |
| Retention time | 4.0 min |
| Limit of detection | 0,1 µg/ml. |
| Column | ODS-2 Hypersil 150 x 4.6 mm 5 µm |

### Equipment for quantification of betaxolol

The HPLC system comprised a Thermo Separations system with Spectrasystems P2000 pump, AS 3000 Auto-injector, SCM 4000 column section and an FL 2000 fluorescence detector. A stock solution of 1000mg/ml betaxolol was frozen (for up to one month) and standards diluted as required, but analysed within 24h (Trocis Product Material Safety Data). The phosphate buffer was 2.56g/l $NaH_2PO_4$, with the pH adjusted to 3.8 with 10% phosphoric acid. Each run was 10 minutes.

(Betaxolol can also be analysed using the thermo HPLC using above conditions and column. The fluorescence detector can be used at excitation 1 228 and emission 1300.) Chromatographic conditions are detailed in Table 2.

Table 2. Chromatographic conditions for detection of betaxolol

| Injection volume | 25 µl |
|---|---|
| Mobile phase | 50% phosphate buffer pH 3.8: 50% acetonitrile |
| Flow rate | 1 ml/min |
| Wavelength | 218 nm |
| Excitation wavelength | 228 nm |
| Emission wavelength | 300 nm |
| Retention time | 3.0 min |
| Limit of detection | 0.1 µg/ml |
| Column | ODS-2 Hypersil 150 x 4.6 mm 5 µm |

### Formulation of latanoprost discs

The prostaglandin analogue latanoprost is an oil which is soluble in acetone and ethanol, but insoluble in water. The commercial preparation is Xalantan and one drop contains 1.5 µg latanoprost. This is therefore the target amount per device. To incorporate the latanoprost, it is dissolved in ethanol and added to the polymer. The ethanol is evaporated under a nitrogen stream and the resultant powder is compressed for 1 minute using a 4 ton compression force in an IR tablet press using a 6 mm die. The discs are left to equilibrate at room temperature before thickness is measured (approx. 0.3 mm). Compression force and dwell time were found to have little impact on the results for polyethylene oxide at the molecular weights studied.

Due to sensitivity problems it was necessary to increase the loading of latanoprost per disc. Average loading per disc of weight approximately 15 mg is 50 µg for PEO 400 (see Table 3). Discs are prepared presently in batches of 6, of which three are used to determine mean load and three for release.

A solution of latanoprost was diluted to 1500mg/ml with ethanol and stored at -70°C. 100 ml per insert was added to the PEO and 10% Carbopol, and the ethanol was evaporated under a stream of nitrogen (about 1h), then left overnight at room temperature to remove any residual ethanol. The PEO and 10% Carbopol were mixed prior to the addition of the latanoprost.

PLGA was ground to a fine powder with a mortar and pestle. Medisorb was allowed to reach room temperature before being ground into a fine powder. Any PLGA inserts were stored in grease proof paper.

PVA was also ground to a finer powder. Betaxolol was mixed with PLGA or PVA as a powder and ground.

Compression of the layers was at 4 tons for 1 min. Anything that was co-compressed had individual layers compressed lighter at 2 tons, then co-compressed at 4 tons. PVA and betaxolol were lightly compressed between PLGA, with PEO underneath, at 2 tons for 1 min, then 1 min at 4 tons, as this layer of the insert may be brittle.

All inserts were allowed to relax for 24h before thickness was measured.

Table 3. Loading of latanoprost into polyethylene oxide discs

|  | Insert weight (mg) | Drug loading ($\mu$g) per insert | Loading (% w/w) |
|---|---|---|---|
| PEO 400 | 15.3.9$\pm$1.67 | 51.17$\pm$ 6.89 | 0.34 |
| PEO 900 | 19.74$\pm$3.54 | 40.95$\pm$8.07 | 0.21 |
| Data is mean $\pm$ standard deviation |  |  |  |

### *Delivery of drug from erodible inserts*

Delivery is monitored in vitro using a Franz-diffusion cell, with the insert in contact with a dialysis membrane to provide support. A membrane with an appropriate molecular weight cut-off is used to allow the passage of pharmaceutical product but not polymer. The receiver solution is an artificial lachrymal fluid, containing NaHCO$_3$ 2.2 g/l; NaCl 6.26 g/l; KC11.79 g/l; CaCl$_2$·2H$_2$O 0.0735 g/l; MgCl$_2$·6H$_2$O 0.0964 g/l in distilled water (pH to 7.5 with 0.1 M HCl). The temperature used in the diffusion cells is controlled so the internal temperature is 32 °C (the temperature of the corneal surface). The tops of the diffusion cells are sealed using parafilm and the system equilibrated overnight (to give a humid environment). The insert is weighed, then placed on the dialysis membrane and resealed. Samples of delivered pharmaceutical product are removed from the receiver fluid and replaced with fresh liquid to maintain a constant volume. Pharmaceutical product in the receiver fluid is analysed by HPLC.

Samples of 1 ml are withdrawn and replaced at given intervals through the side arm. The insert is placed in the donor at time 0.

Samples were analysed using HPLC methods described above. It is recommended that betaxolol is analysed within 24h, or samples frozen. The quantity of drug which has been released at any given time can be calculated using the following equation:

$$M_t[n] = \frac{V_r.C[n] + V_s \sum_{m=1}^{n-1} C[m]}{1000}$$

wherein:

V$_r$ is the volume of receiver (ml)
V$_s$ the sample volume
C[n] is the concentration in the dissolution medium (mg/l)
$\Sigma$C[m] is the sum total of previous concentrations (mg/l)
M$_t$ is the drug released at time t (mg)

Figure 2 shows a release profile of of latanoprost with 10% Carbopol in PEO having a molecular weight of 400,000 and betaxolol with PVA which is sandwiched between two layers of PGLA and being supported. Similarly, Figure 3 shows a release profile of latanoprost and timolol in an identical system.

25mg of PEO having a molecular weight of 400,000 containing latanoprost was added to the sandwich of 15mg PLGA (having a molecular weight of less than 5,000), 15mg PVA containing betaxolol and 15mg PLGA. The inserts were supported on the sides. The inserts weighed 56 $\pm$ 4mg, were 3.32 $\pm$ 0.18mm thick, and contained 59 $\pm$ 3$\mu$g latanoprost and 47 $\pm$ 3$\mu$g betaxolol.

A bioadhesive polymer may be used in aiding the retention of disc at the application site, although the discs themselves are adhesive. The bioadhesion can be measured *in vitro.* Suitable polymers include carrageenan, collagen, HPC, gelatin, and polyacrylic acid (Carbopol).

*Formulation of delayed release component*

The delivery of the beta-blocker betaxolol from PLGA discs (50:50 ratio but using a range of molecular weights) has also been investigated. The discs are preferably formulated in the same way as the erodible component, although formulation of the discs can be modified in any obvious manner which still allows the formation of the discs. For example, this can be by making microspheres, which can then be compressed or embedded in a matrix, or the particles can be coated with a semi-permeable polymer coating such as cellulose acetate or a high viscosity polymer such as HPMC. Optionally, osmotically active agents can be incorporated to rupture the system and provide a faster rate of delivery once the initial lag period has been attained, and one side of the insert can be coated with a polypropylene support.

In another design, the drug (beta-blocker) is added to PVA (9,000 to 10,000 molecular weight, 80% hydrolysed) and this is layered between the two discs of low molecular weight PLGA.

**Travoprost/Timolol System**

The experiments described above for the latanoprost/betaxolol system were repeated using travoprost as the prostaglandin analogue and timolol maleate (herein referred to as "timolol") as the beta-blocker.

The travoprost was analysed using the hplc method described above for latanoprost.

Inserts were prepared using travoprost alone and also travoprost with timolol. Travoprost was loaded into PEO inserts containing 10% carbopol and compressed at 2 tons.

Loading was $6.59\pm0.08$ μg/mg and insert weight was $18.35 \pm 0.85$ mg. The thickness of these single layer inserts was $0.74 \pm 0.05$ mm.

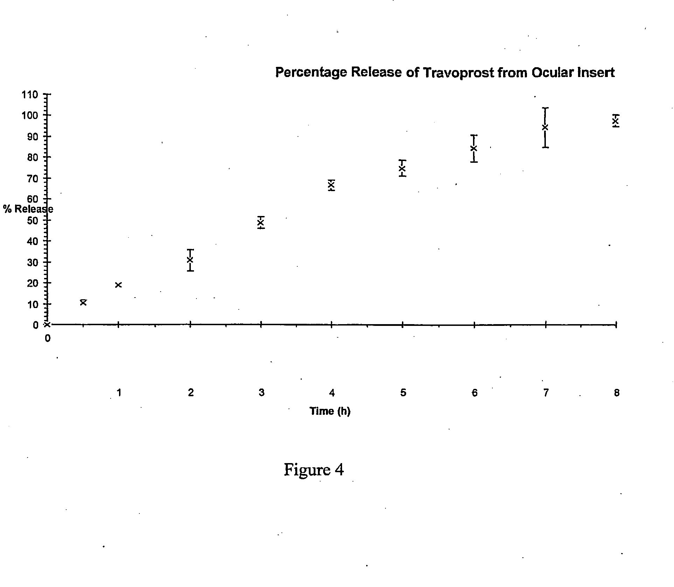

Release was studied using the humidified Franz-diffusion cell described above for latanoprost. Results showed that travoprost was slowly released from the PEO-carbopol matrix at a substantially-constant rate over a period of about 8 hours, as shown in Figure 4.

A laminated insert was then prepared using travoprost and timolol as described above for the latanoprost/betaxolol system

The insert components were lightly precompressed at 1 ton then compressed together to form the laminate at 2 tons.

Loading was $2.09 \pm 0.10$ μg/mg for timolol and $2.60 \pm 0.19$ μg/mg for travoprost. Insert weight was $54.77\pm 2.61$ mg. The thickness of these single layer inserts was $2.27 \pm 0.15$ mm.

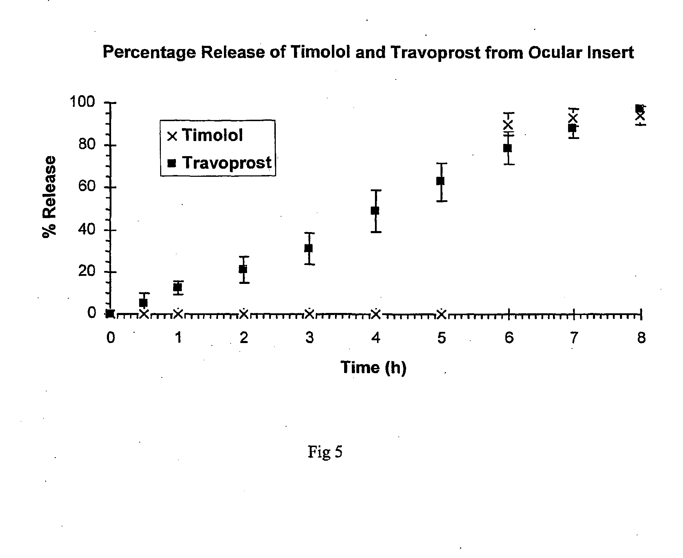

Release was studied using the humidified Franz-diffusion cell as described above and results showed that the insert released this new combination of drugs (i.e. travoprost and timolol) in a similar manner to latanoprost and betaxolol, as shown in Fig 5.

Whilst the travoprost was released at a substantially constant rate over a period of about 8 hours, essentially no timolol was released for the first 5 hours, but over 90% of the timolol had been released by the time 6 hours had elapsed from the beginning of the experiment.

*Effect of different conditions upon drug delivery*

All of the above experiments in relation to the present invention were carried out at humidity at 32°C, in order to recreate as far as possible the conditions of the eye. However, the delivery system of the invention has also been tested under a number of different conditions; when immersed in lachrymal fluid, when washed with lachrymal fluid, and at 37°C, i.e. body temperature. It was shown that in each of the different conditions, the delivery of the prostaglandin, and the beta-blocker when released, was even more rapid than under the conditions of humidity at 32°C.

**Claims**

**1.** A delivery system incorporating pharmaceutical products for delivery to an eye in the treatment of primary open-angle glaucoma (POAG), comprising:

(a) a biocompatible erodible material incorporating a therapeutically-effective amount of a prostaglandin analogue, and
(b) a reservoir containing a therapeutically-effective amount of a beta-blocker, whereby, when the delivery system is placed in the eye the prostaglandin analogue is delivered gradually as the erodible material is eroded, and the beta-blocker is delivered rapidly when at least a predetermined portion of the erodible material has been eroded.

**2.** A delivery system according to claim 1, wherein the prostaglandin analogue and beta-blocker are located in separate layers.

**3.** A delivery system according to claim 1 or claim 2, wherein the rate of erosion of the erodible material is such that the erosion is completed during periods of high intraocular pressure and low ocular blood flow.

**4.** A delivery system according to any preceding claim, further comprising a layer of a biodegradable polymer separating the erodible material incorporating the prostaglandin analogue and the reservoir containing the beta-blocker, wherein at least a predetermined portion of the biodegradable polymer is biodegraded prior to the delivery of the beta-blocker.

**5.** A delivery system according to any preceding claim, wherein the reservoir containing the beta-blocker has upper, lower and side surfaces, and the or each side surface is sealed with an insulating support to inhibit premature leakage of the beta-blocker.

**6.** A delivery system according to claim 5, further comprising a barrier layer of a biodegradable polymer or a non-biodegradable polymer located on the lower surface of the reservoir containing the beta-blocker while the erodible material incorporating the prostaglandin analogue is located on the upper surface of the reservoir, or vice versa.

**7.** A delivery system according to claim 2, wherein the erodible material comprises a semi-permeable membrane to delay the delivery of the beta-blocker.

**8.** A delivery system according to any preceding claim, wherein the beta-blocker is delivered when substantially all of the erodible material has been eroded.

**9.** A delivery system according to any preceding claim, wherein the erodible material comprises a polymeric material.

**10.** A delivery system according to claim 9, wherein the polymeric material is selected from polyethylene oxide (PEO), poly(alkyl) cyanoacrylates, and co-polymers of methyl vinyl ether and maleic anhydride, poly(alkyl cyanoacrylates), copolymers of polylactic and glycolic acid, or copolymers of polyethylene oxide and a methacrylic acid polymer.

**11.** A delivery system according to any preceding claim, wherein the prostaglandin analogue is selected from latanoprost, bimatoprost, travoprost and unoprostone isopropyl.

**12.** A delivery system according to any preceding claim, wherein the reservoir comprises a material selected from glass, metal, ceramics, polyvinyl alcohol (PVA), cross-linked polyvinyl alcohol, polyvinyl acetate, polyvinylbutyrate, cross-linked polyvinyl butyrate, ethylene ethylacrylate copolymer, polyethyl hexylacrylate, polyvinyl chloride, polyvinyl acetals, plasticised ethylene vinylacetate copolymer, ethylene vinylchloride copolymer, polyvinyl esters, polyvinylformal, polyamides, polymethylmethacrylate, polybutylmethacrylate, plasticised polyvinyl chloride, plasticised nylon, plasticised soft nylon, plasticised polyethylene terephthalate, natural rubber, polyisoprene, polyisobutylene, polybutadiene, polyethylene, polytetrafluoroethylene, polyvinylidene chloride, polyacrylonitrile, cross-linked polyvinylpyrrolidone, polytrifluorochloroethylene, chlorinated polyethylene, poly(1,4'-isopropylidene diphenylene carbonate), vinylidene chloride, acrylonitrile copolymer, vinyl chloride-diethyl fumerale copolymer, butadiene/styrene copolymers, silicone rubbers, medical grade polydimethylsiloxanes, ethylene-propylene rubber, silicone-carbonate copolymers, vinylidene chloride-vinyl chloride copolymer, vinyl chloride-acrylonitrile copolymer and vinylidene chloride-acrylonitride copolymer; non-biodegradable polymers such as polymethylmethacrylate, a silicone elastomer, or silicone rubber, polyolefins, homopolymers, and copolymers of vinyl acetate, polyvinylchlorides, homopolymers and copolymers of acrylates, polyurethanes, polyvinylpyrrolidone, 2-pyrrolidone, polyacrylonitrile butadiene, polycarbonates, polyamides, fluoropolymers, polystyrenes, homopolymers and copolymers of styrene acrylonitrile, cellulose acetate, homopolymers and copolymers of acrylonitrile butadiene styrene, polymethylpentene, polysulfones, polyesters, polyimides, natural rubber, polyisobutylene and polymethylstyrene; and synthetic biodegradable polymers such as polyesters of molecular weight from about 4,000 to about 100,000, homopolymers and copolymers of (poly)lactic acid and (poly)glycolic acid, such as polylactic glycolic acid copolymer (PLGA), polycaprolactone, homopolymers and copolymers of polyanhydrides, bis(p-anhydride) and poly(p-carboxyphenoxy) alkyl, homopolymers and copolymers of dicarboxylic acids, polymeric fatty acid dimer compounds, poly(alkyl-2-cyanoacrylate) such as poly(hexyl-2-cyanoacrylate), collagen (gelatin), polyacetals, divinyloxyalkylenes, polydihydropyrans, polyphosphazenes, homopolymers and copolymers of amino acids, polydioxinones, polyalkylcyano acetates, polysaccarides and their derivatives, cellulose and hydroxymethyl cellulose.

**13.** A delivery system according to any preceding claim, wherein the beta-blocker is betaxolol or a salt thereof, or carteolol hydrochloride, levobunolol hydrochloride, metipranolol, or timolol maleate.

**14.** A delivery system according to claim 4, wherein the biodegradable polymer is polylactic glycolic acid copolymer (PLGA).

**15.** A delivery system according to claim 6, wherein the barrier layer comprises either PLGA or polypropylene.

**16.** A delivery system according to any preceding claim, wherein the erodible material further comprises a mucoadhesive polymer.

**17.** A delivery system incorporating pharmaceutical products for delivery to an eye in the treatment of primary open-angle glaucoma (POAG), comprising:

(a) a layer of PEO incorporating a therapeutically-effective amount of latanoprost;
(b) a layer of PVA containing a therapeutically-effective amount ofbetaxolol;
(c) a layer of PLGA separating the PEO and PVA layers;
(d) a polypropylene insulating support located on the side surface(s) of the PVA layer;
(e) a barrier layer of PLGA or polypropylene located on the lower surface of the reservoir containing the betaxolol while the layer of PEO incorporating the latanoprost is located on the upper surface of the layer of PVA, or vice versa;
(f) a polyacrylic acid mucoadhesive polymer,

whereby, when the delivery system is placed in the eye the latanoprost is delivered gradually as the PEO is eroded, and the betaxolol is delivered rapidly when at least a predetermined portion of the PEO has been eroded.

**18.** Use of a delivery system according to any one of claims 1 to 17 for the manufacture of a medicament for treating or preventing primary open-angle glaucoma in a mammal, comprising administering to the mammal a therapeutic amount of one or more prostaglandin analogues or beta-blockers

**19.** Use of a delivery system according to any one of claims 1 to 17 for the manufacture of a medicament in the treatment of primary open-angle glaucoma.

**20.** A process for the preparation of a delivery system according to any one of claims 1 to 17, comprising the steps of:

(a) dissolving a formulation containing the prostaglandin analogue in a solvent;
(b) adding this solution to the erodible material;
(c) removing the solvent;
(d) compressing the resultant powder into discs;
(e) dissolving a formulation containing the beta-blocker in a solvent;
(f) adding this solution to the reservoir;
(g) removing the solvent;
(h) compressing the resultant powder into discs;
(i) combining discs from steps (d) and (h).

**21.** A process according to claim 20, further comprising the step of incorporating a layer of biodegradable polymer separating the erodible material incorporating the prostaglandin analogue and the reservoir containing the beta-blocker.

**Patentansprüche**

**1.** Abgabesystem, das pharmazeutische Produkte zur Abgabe an ein Auge bei der Behandlung eines primären Weitwinkelglaukoms (Primary Open-Angle Glaucoma POAG) einschließt und das

(a) ein biokompatibles erodierbares Material, das eine therapeutisch wirksame Menge eines Prostaglandin-Analogs einschließt, und
(b) einen Speicher aufweist, der eine therapeutisch wirksame Menge eines Beta-Blockers enthält,

- wobei, wenn das Abgabesystem in dem Auge platziert ist, das Prostaglandin-Analog allmählich abgegeben wird, wenn das erodierbare Material erodiert wird, und
- wobei der Betablocker schnell abgegeben wird, wenn wenigstens ein vorgegebener Teil des erodierbaren Materials erodiert worden ist.

2. Abgabesystem nach Anspruch 1, bei welchem das Prostaglandin-Analog und der Betablocker in getrennten Schichten angeordnet sind.

3. Abgabesystem nach Anspruch 1 oder Anspruch 2, bei welchem die Erosionsrate des erodierbaren Materials so beschaffen ist, dass die Erosion während Perioden mit hohem Augeninnendruck und niedriger Augendurchblutung abgeschlossen wird.

4. Abgabesystem nach einem vorhergehenden Anspruch, welches weiterhin eine Schicht aus einem biologisch abbaubaren Polymer aufweist, welche das das Prostaglandin-Analog einschließende erodierbare Material und den den Betablocker enthaltenden Speicher trennt, wobei wenigstens ein vorgegebener Teil des biologisch abbaubaren Polymers vor der Abgabe des Betablockers biologisch abgebaut wird.

5. Abgabesystem nach einem vorhergehenden Anspruch, bei welchem der den Betablocker enthaltende Speicher obere, untere und Seitenflächen aufweist und die oder jede Seitenfläche mit einem Isolierträger abgedichtet ist, um einen vorzeitigen Abgang des Betablockers zu unterbinden.

6. Abgabesystem nach Anspruch 5, welches weiterhin eine Sperrschicht aus einem biologisch abbaubaren Polymer oder einem biologisch nicht abbaubaren Polymer aufweist, die auf der unteren Oberfläche des den Betablocker enthaltenden Speichers angeordnet ist, während sich das das Prostaglandin-Analog einschließende erodierbare Material auf der oberen Oberfläche des Speichers befindet oder umgekehrt.

7. Abgabesystem nach Anspruch 2, bei welchem das erodierbare Material eine semipermeable Membran aufweist, um die Abgabe des Betablockers zu verzögern.

8. Abgabesystem nach einem vorhergehenden Anspruch, bei welchem der Betablocker abgegeben wird, wenn im Wesentlichen das ganze erodierbare Material erodiert worden ist.

9. Abgabesystem nach einem vorhergehenden Anspruch, bei welchem das erodierbare Material ein polymeres Material aufweist.

10. Abgabesystem nach Anspruch 9, bei welchem das polymere Material aus Polyethylenoxid (PEO), Poly(alkyl-)Cyanoacrylaten und Copolymeren von Methylvinylether und Maleinsäureanhydrid, Poly(alkylcyanoacrylaten), Copolymeren von Polymilchsäure und Glykolsäure oder Copolymeren von Polyethylenoxid und einem Methacrylsäurepolymer ausgewählt wird.

11. Abgabesystem nach einem vorhergehenden Anspruch, bei welchem das Prostaglandin-Analog aus Latanoprost, Bimatoprost, Travoprost und Unoproston-Isopropyl ausgewählt wird.

12. Abgabesystem nach einem vorhergehenden Anspruch, bei welchem der Speicher ein Material aufweist, das aus ausgewählt wird aus Glas, Metall, Keramik, Polyvinylalkohol (PVA), vernetztem Polyvinylalkohol, Polyvinylacetat, Polyvinylbutyrat, vernetztem Polyvinylbutyrat, Ethylenethylacrylat-Copolymer, Polyethylhexylacrylat, Polyvinylchlorid, Polyvinylacetalen, plastifiziertem Ethylenvinylacetat-Copolymer, Ethylenvinylchlorid-Copolymer, Polyvinylestem, Polyvinylformal, Polyamiden, Polymethylmethacrylat, Polybutylmethacrylat; plastifiziertem Polyvinylchlorid, plastifiziertem Nylon, plastifiziertem Weichnylon, plastifiziertem Polyethylenterephthalat, Naturkautschuk, Polyisopren, Polyisobutylen, Polybutadien, Polyethylen, Polytetrafluorethylen, Polyvinylidenchlorid, Polyacrylnitril, vernetztem Polyvinylpyrrolidon, Polytrifluorchlorethylen, chloriertem Polyethylen, Poly(1,4'-Isopropyliden-Diphenylen-Carbonat), Vinylidenchlorid, Acrylnitril-Copolymer, Vinylchlorid-Diethylfumeral-Copolymer, Butadien/Styrol-Copolymeren, Siliconkautschuken, Polydimethylsiloxanen mit medizinischem Gütegrad, Ethylen-Propylen-Kautschuk, Silicon-Carbonat-Copolymere, Vinylidenchlorid-Vinylchlorid-Copolymere, Vinylchlorid-Acrylnitril-Copolymer und Vinylidenchloride-Acrylnitrid-Copolymer; biologisch nicht abbaubare Polymere, wie Polymethylmethacrylat, ein Siliconelastomer oder Siliconkautschuk, Polyolefinen, Homopolymeren und Copolymeren von Vinylacetat, Polyvinylchloriden, Homopolymeren und Copolymeren von Acrylaten, Polyurethanen, Polyvinylpyrrolidon, 2-Pyrrolidon, Polyacrylnitrilbutadien, Polycarbonaten, Polyamiden, Fluorpolymeren, Polystyrolen, Homopolymeren und Copolymeren von Styrolacrylnitril, Celluloseacetat, Homopolymeren und Copolymeren von Acrylnitrilbutadienstyrol, Polymethylpenten, Polysulfonen, Polyestern, Polyimiden, Naturkautschuk, Polyisobutylen und Polymethylstyrol; und synthetische biologisch abbaubare Polymere, wie Polyester mit einem Molekulargewicht von etwa 4.000 bis etwa 100.000, Homopolymeren und Copolymeren von (Poly)Milchsäure und (Poly)Glycolsäure, wie Polymilchsäure-Glycolsäure-Copolymer (PLGA), Polycaprolacton, Homopolymeren und Copolymeren von Polyanhydriden, bis(p-Anhydrid)- und Poly (p-Carboxyphenoxy)-Alkyl, Homopolymeren und Copolymeren von Dicarbonsäuren, polymere Fettsäure-Dimer-Verbindungen, Poly(Alkyl-2-Cyanoacrylat), wie Poly(Hexyl-2-Cyanoacrylat), Collagen (Gelatine), Polyacetalen, Divinyloxyalkylenen, Polydihydropyranen, Polyphosphazenen, Homopolymeren und Copolymeren von Aminosäuren, Polydioxinonen, Polyalkylcyanoacetaten, Polysacchariden und ihren Derivaten, Zellulose und Hydroxymethylzellulose.

13. Abgabesystem nach einem vorhergehenden Anspruch, bei welchem der Betablocker Betaxolol oder ein Salz davon oder Carteololhydrochlorid, Levobunololhydrochlorid, Metipranolol oder Timololmaleat ist.

14. Abgabesystem nach Anspruch 4, bei welchem das biologisch zersetzbare Polymer ein Polymilchsäureglycolsäure-Copolymer (PLGA) ist.

15. Abgabesystem nach Anspruch 6, bei welchem die Sperrschicht entweder PLGA oder Polypropylen aufweist.

16. Abgabesystem nach einem vorhergehenden Anspruch, bei welchem das erodierbare Material weiterhin ein mucoadhäsives Polymer aufweist.

17. Abgabesystem, das pharmazeutische Produkte zur Abgabe an ein Auge bei der Behandlung eines primären Weitwinkelglaukoms (POAG) einschließt und das

   (a) eine eine therapeutisch wirksame Menge von Latanoprost einschließende Schicht aus PEO;
   (b) eine eine therapeutisch wirksame Menge von Betaxolol enthaltende Schicht aus PVA;
   (c) eine die PEO- und die PVA-Schicht trennende Schicht aus PLGA;
   (d) einen Polypropylen-Isolierträger, der sich an der/den Seitenfläche(n) der PVA-Schicht befindet,
   (e) eine Sperrschicht aus PLGA oder Polypropylen, die sich auf der unteren Oberfläche des das Betaxolol enthaltenden Speichers befindet, während sich die das Latanoprost einschließende PEO-Schicht auf der oberen Oberfläche der PVA-Schicht befindet oder umgekehrt, und
   (f) ein mucoadhäsives Polyacrylsäurepolymer aufweist,

      - wobei, wenn das Abgabesystem in dem Auge platziert ist, das Latanoprost allmählich abgegeben wird, wenn das PEO erodiert wird, und
      - wobei das Betaxolol schnell abgegeben wird, wenn wenigstens ein vorgegebener Teil des PEO erodiert worden ist.

18. Verwendung eines Abgabesystems nach einem der Ansprüche 1 bis 17 zur Herstellung eines Medikaments für die Behandlung oder Prävention eines primären Weitwinkelglaukoms bei einem Säuger, wobei dem Säuger eine therapeutische Menge von einem oder mehreren Prostaglandin-Analogen oder Betablockern verabreicht wird.

19. Verwendung eines Abgabesystems nach einem der Ansprüche 1 bis 17 für die Herstellung eines Medikaments bei der Behandlung eines primären Weitwinkelglaukoms.

20. Verfahren zur Herstellung eines Abgabesystems nach einem der Ansprüche 1 bis 17, welches die Schritte aufweist:

   (a) Auflösen eines Ansatzes, der das Prostaglandin-Analog in einem Lösungsmittel enthält;
   (b) Zugeben dieser Lösung zu dem erodierbaren Material;
   (c) Entfernen des Lösungsmittels;
   (d) Komprimieren des erhaltenen Pulvers zu Scheiben;
   (e) Auflösen eines Ansatzes, der den Betablocker in einem Lösungsmittel enthält;
   (f) Zugeben dieser Lösung zu dem Speicher;
   (g) Entfernen des Lösungsmittels;
   (h) Komprimieren des erhaltenen Pulvers zu Scheiben und
   (i) Zusammenfügen der Scheiben aus den Schritten (d) und (h).

**21.** Verfahren nach Anspruch 20, welches weiterhin den Schritt aufweist, eine Schicht eines biologisch abbaubaren Polymers einzuschließen, die das das Prostaglandin-Analog einschließende erodierbare Material und den den Betablocker enthaltenden Speicher trennt.

**Revendications**

**1.** Système d'administration comprenant des produits pharmaceutiques prévus pour être administrés à un oeil pour le traitement d'un glaucome primaire à angle ouvert (GPAO), comportant :

(a) une matière érodable biocompatible comprenant une quantité efficace sur le plan thérapeutique d'un analogue de prostaglandine, et
(b) un réservoir contenant une quantité efficace du point de vue thérapeutique d'un bêtabloquant,

par lequel, lorsque le système d'administration est placé dans l'oeil, l'analogue de prostaglandine est administré graduellement pendant que la matière érodable est érodée, et le bêtabloquant est administré rapidement lorsqu'au moins une partie prédéterminée de la matière érodable a été érodée.

**2.** Système d'administration selon la revendication 1, dans lequel l'analogue de prostaglandine et le bêtabloquant sont situés dans des couches distinctes.

**3.** Système d'administration selon la revendication 1 ou la revendication 2, dans lequel la vitesse d'érosion de la matière érodable est telle que l'érosion est achevée au cours de périodes de forte pression intraoculaire et de faible débit sanguin oculaire.

**4.** Système d'administration selon l'une quelconque des revendications précédentes, comportant en outre une couche de polymère biodégradable séparant la matière érodable contenant l'analogue de prostaglandine et le réservoir contenant le bêtabloquant, dans lequel au moins une partie prédéterminée du polymère biodégradable est biodégradée avant l'administration du bêtabloquant.

**5.** Système d'administration selon l'une quelconque des revendications précédentes, dans lequel le réservoir contenant le bêtabloquant présente des faces supérieure, inférieure et latérales, et dans lequel la ou chaque face latérale est scellée avec un support isolant pour empêcher toute fuite prématurée du bêtabloquant.

**6.** Système d'administration selon la revendication 5, comportant en outre une couche-barrière d'un polymère biodégradable ou d'un polymère non-biodégradable située sur la face inférieure du réservoir contenant le bêtabloquant alors que la matière érodable contenant l'analogue de prostaglandine est située sur la face supérieure du réservoir, ou vice versa.

**7.** Système d'administration selon la revendication 2, dans lequel la matière érodable comporte une membrane semi-perméable pour retarder l'administration du bêtabloquant.

**8.** Système d'administration selon l'une quelconque des revendications précédentes, dans lequel le bêtabloquant est administré lorsque sensiblement toute la matière érodable a été érodée.

**9.** Système d'administration selon l'une quelconque des revendications précédentes, dans lequel la matière érodable comporte une matière polymère.

**10.** Système d'administration selon la revendication 9, dans lequel la matière polymère est choisie parmi l'oxyde de polyéthylène (PEO), les poly(alkyl)cyanoacrylates, et les copolymères d'éther méthylique de vinyle et d'anhydride maléique, les poly(alkyl)cyanoacrylates, les copolymères d'acide polylactique et glycolique, ou les copolymères d'oxyde de polyéthylène et d' un polymère d'acide méthacrylique.

**11.** Système d'administration selon l'une quelconque des revendications précédentes, dans lequel l'analogue de prostaglandine est choisi parmi le latanoprost, le bimatoprost, le travoprost et l'isopropyle d'unoprostone.

**12.** Système d'administration selon l'une quelconque des revendications précédentes, dans lequel le réservoir comporte une matière choisie parmi le verre, le métal, la céramique, l'alcool de polyvinyle (PVA), l'alcool de polyvinyle réticulé, l'acétate de polyvinyle, le polyvinylbutyrate, le butyrate de polyvinyle réticulé, le copolymère d'éthylacrylate d'éthylène, l'hexylacrylate de polyéthyle, le chlorure de polyvinyle, les acétals de polyvinyle, le copolymère de vinylacetate d'éthylène plastifié, le copolymère de vinylchlorure d'éthylène, les esters de polyvinyle, le polyvinylformal, les polyamides, le polyméthylméthacrylate, le polybutylméthacrylate, le chlorure de polyvinyle plastifié, le nylon plastifié, le nylon mou plastifié, le polyéthylène téréphtalate plastifié, le caoutchouc naturel, le polyisoprène, le polyisobutylène, le polybutadiène, le polyéthylène, le polytétrafluoroéthylène, le chlorure de polyvinylidène, le polyacrylonitrile, la polyvinylpyrrolidone réticulée, le polytrifluorochloroéthylène, le polyéthylène chloré, le poly(1,4'-carbonate de diphenylène isopropylidène), le chlorure de vinylidène, le copolymère d'acrylonitrile, le copolymère de chlorure-diéthyle de vinyle fumant, les copolymères de butadiène/styrène, les caoutchoucs de silicone, les polydiméthylsiloxanes de catégorie médicale, le caoutchouc d'éthylène-propylène, les copolymères de silicone-carbonate, le copolymère de chlorure de vinyle-chlorure de vinylidène, le copolymère de chlorure de vinyle -acrylonitrile et le copolymère de chlorure-acrylonitrure de vinylidène ; les polymères non-biodégradables tels que le polyméthylméthacrylate, un élastomère de silicone un caoutchouc de silicone, les polyoléfines, les homopolymères, et les copolymères d'acétate de vinyle, les polyvinylchlorures, homopolymères et copolymères d'acrylates, les polyuréthanes, la polyvinylpyrrolidone, la 2-pyrrolidone, le polyacrylonitrile-butadiène, les polycarbonates, les polyamides, les fluoropolymères, les polystyrènes, les homopolymères et les copolymères de l'acrylonitrile-styrène, l'acétate de cellulose, les homopolymères et copolymères de l'acrylonitrile-styrène-butadiène, le polyméthylpentène, les polysulfones, les polyester, les polyimides, le caoutchouc naturel, le polyisobutylène et le polyméthylstyrène ; et les polymères biodégradables synthétiques tels que les polyesters de poids moléculaire compris entre environ 4.000 et environ 100.000, les homopolymères et les copolymères de l'acide (poly)lactique et de l'acide (poly)glycolique, tel que le copolymère d'acide glycolique - polylactique (PLGA), la polycaprolactone, les homopolymères et copolymères des polyanhydrides, le bis(p-anhydride) et poly(p-carboxyphenoxy) d'alkyle, les homopolymères et les copolymères d'acides dicarboxyliques, les composés polymères de dimère d'acide gras, le poly(alkyl-2-cyanoacrylate) tel que le poly(hexyl-2-cyanoacrylate), le collagène (gélatine), les polyacétals, les divinyloxyalkylènes, les polydihydropyranes, les polyphosphazènes, les homopolymères et copolymères des acides aminés, les polydioxinones, les polyalkyl-cyano-acétates, les polysaccharides et leurs dérivés, la cellulose et la cellulose hydroxyméthylique.

13. Système d'administration selon l'une quelconque des revendications précédentes, dans lequel le bêtabloquant est du bétaxolol ou un sel de celui-ci, ou du chlorhydrate de cartéolol, du chlorhydrate de lévobunolol, du métipranolol ou du maléate de timolol.

14. Système d'administration selon la revendication 4, dans lequel le polymère biodégradable est un copolymère d'acide glycolique et polylactique (PLGA).

15. Système d'administration selon la revendication 6, dans lequel la couche-barrière comporte du PLGA ou du polypropylène.

16. Système d'administration selon l'une quelconque des revendications précédentes, dans lequel la matière érodable comprend en outre un polymère muco-adhésif.

17. Système d'administration comprenant des produits pharmaceutiques prévus pour être administrés à un oeil dans le traitement du glaucome primaire à angle ouvert (GPAO), comportant :

(a) une couche de PEO comprenant une quantité efficace sur le plan thérapeutique de latanoprost ;
(b) une couche de PVA contenant une quantité efficace sur le plan thérapeutique de betaxolol ;
(c) une couche de PLGA séparant les couches de PEO et de PVA ;
(d) un support isolant de polypropylène situé sur la(les) face(s) latérale(s) de la couche de PVA ;
(e) une couche-barrière de PLGA ou de polypropylène située sur la face inférieure du réservoir contenant le bétaxolol tandis que la couche de PEO comprenant le latanoprost est située sur la face supérieure de la couche de PVA, ou vice versa ;
(f) un polymère d'acide polyacrylique muco-adhésif,

selon lequel, lorsque le système d'administration est placé dans l'oeil, le latanoprost est administré graduellement pendant que le PEO est érodé, et le bétaxolol est administré rapidement lorsqu'au moins une partie prédéterminée du PEO a été érodée.

18. Utilisation d'un système d'administration selon l'une quelconque des revendications 1 à 17 pour la fabrication d'un médicament pour le traitement ou la prévention du glaucome primaire à angle ouvert chez un mammifère, comprenant l'administration au mammifère d'une quantité thérapeutique d'un ou plusieurs analogues de prostaglandine ou des bêtabloquants.

**19.** Utilisation d'un système d'administration selon l'une quelconque des revendications 1 à 17 pour la fabrication d'un médicament pour le traitement du glaucome primaire à angle ouvert.

**20.** Procédé pour la préparation d'un système d'administration selon l'une quelconque des revendications 1 à 17, comportant les étapes suivantes :

(a) dissolution d'une formulation contenant l'analogue de prostaglandine dans un solvant ;
(b) ajout de cette solution à la matière érodable ;
(c) élimination du solvant ;
(d) compression en disques de la poudre qui en résulte ;
(e) dissolution d'une formulation contenant le bêtabloquant dans un solvant ;
(f) ajout de cette solution dans le réservoir ;
(g) élimination du solvant ;
(h) compression en disques de la poudre qui en résulte ;
(i) combinaison des disques des étapes (d) et (h).

**21.** Procédé selon la revendication 20, comportant en outre l'étape d'incorporation d'une couche de polymère biodégradable séparant la matière érodable comprenant l'analogue de prostaglandine et le réservoir contenant le bêtabloquant.

FIGURE 1

Figure 2 Percentage release of betaxolol and latanoprost from ocular insert

Figure 3 Percentage release of timolol and latanoprost from ocular insert

**Percentage Release of Travoprost from Ocular Insert**

Figure 4

**Percentage Release of Timolol and Travoprost from Ocular Insert**

Fig 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2004013704 A

- US 6051576 A